# EUROPEAN PATENT APPLICATION

(11) **EP 1 275 394 A2**
(43) Date of publication of application: **15.01.2003**
(21) Application number: 02254789.7
(22) Date of filing: 09.07.2002
(51) Int. Cl.: A61K 31/565

(54) **Ultra low dose oral contraceptives with sustained efficacy that induces amenorrhea**

(30) Priority: 10.07.2001 US 902389
(71) Applicant: MEDICAL COLLEGE OF HAMPTON ROADS, Norfolk, VA 23507 (US)
(72) Inventor: Hodgen, Gary D., Virginia Beach, Virginia 23455 (US); Anderson, Freedolph D., Virginia Beach, Virginia 23455 (US); Williams, Robert F., Norfolk, Virginia 23503 (US)
(74) Representative: Samuels, Lucy Alice

(57) **Abstract**

A method of female contraception involves administering a combination of estrogen and progestin continuously for more than a year in which the daily amounts of estrogen and progestin are equivalent to about 5-35 mcg of ethinyl estradiol and about 0.025 to 10 mg of norethindrone acetate, respectively. The advantages include lack of menstrual bleeding, less patient anemia, less total exposure to medication when compared to a 35 microgram (low dose) containing oral contraceptive, reduced risk of endometrial cancer, higher compliance rates and more lifestyle convenience for patients who desire less uterine bleeding each year or longer.

## Description

### BACKGROUND OF THE INVENTION

The ovarian/menstrual cycle is a complex event characterized by an estrogen rich follicular phase and, after ovulation, a progesterone rich luteal phase. Each has a duration of approximately 14 days resulting in an intermenstrual interval of about 28 days. The endometrial tissue responds to the changes in hormonal milieu.

The onset of menstruation is the beginning of a new menstrual cycle and is counted as day 1. During a span of about 5 to 7 days, the superficial layers of the endometrium, which grew and developed during the antecedent ovarian/menstrual cycle, are sloughed because demise of the extant corpus luteum in the non-fertile menstrual cycle is associated with a loss of progesterone secretion. Ovarian follicular maturation occurs progressively resulting in a rise in the circulating levels of estrogen, which in turn leads to new endometrial proliferation.

The dominant ovarian follicle undergoes ovulation near mid-cycle, generally between menstrual cycle days 12 to 16 and is converted from a predominantly estrogen source to a predominantly progesterone source (the corpus luteum). The increasing level of progesterone in the blood converts the proliferative endometrium to a secretory phase in which the tissue proliferation has promptly abated, leading to the formation of endometrial glands or organs. When the ovulated oocyte is viably fertilized and continues its progressive embryonic cleavage, the secretory endometrium and the conceptus can interact to bring about implantation (nidation), beginning about 6 to 8 days after fertilization.

If an ongoing pregnancy is to be established via implantation, the embryo will attach and burrow into the secretory endometrium and begin to produce human chorionic gonadotropin (HCG). The HCG in turn stimulates extended corpus luteum function, i.e. the progesterone production remains elevated, and, in turn, menses does not occur in the fertile menstrual cycle, Pregnancy, typically, can then be established.

In the non-fertile menstrual cycle, the waning level of progesterone in the blood causes the endometrial tissue to be sloughed. This starts a subsequent menstrual cycle.

Because endometrial proliferation serves to prepare the uterus for an impending pregnancy, manipulation of hormones and of the uterine environment can provide contraception. For example, estrogens are known to decrease follicle stimulating hormone secretion by feedback inhibition. Under certain circumstances, estrogens can also inhibit luteinizing hormone secretion, once again by negative feedback. Under normal circumstances, the spike of circulating estrogen found just prior to ovulation induces the surge of gonadotropic hormones that occurs just prior to and resulting in ovulation. High doses of estrogen immediately post-coitally also can prevent conception probably due to interference with implantation.

Progestins can also provide contraception. Endogenous progesterone after estrogen is responsible for the progestational changes of the endometrium and the cyclic changes of cells and tissue in the cervix and the vagina. Administration of progestin makes the cervical mucus thick, tenacious and cellular which is believed to impede spermatozoal transport. Administration of progestin also inhibits luteinizing hormone secretion and blocks ovulation in humans.

The most prevalent form of oral contraception is a pill that combines both an estrogen and a progestin, a so-called combined oral contraceptive preparation.

Alternatively, there are contraceptive preparations that comprise progestin only, However, the progestin-only preparations have a more varied spectrum of side effects than do the combined preparations, especially more breakthrough bleeding. As a result, the combined preparations are the preferred oral contraceptives in use today (Sheth et al., *Contraception* 25:243, 1982).

Whereas the conventional 21 day pill packs with a 7 day "pill free" or placebo interval worked well when oral contraceptives were of higher dosage, as the doses have come down, for both the estrogen and progestin components, bleeding problems have increased in frequency, especially in the early months of oral contraceptive use, but even persistently so in some patients. Since the advent of combined estrogen-progestin medications as oral contraceptives, there has been a steady downward adjustment of the daily estrogen dosage. Concurrently, where exposure to the progestin component has also been lowered, reduced androgenicity has remained an ongoing priority. Together these adaptations in formulation have been presented in a variety of regimens, both monophasic and multiphasic. Each have their own advantages and disadvantages. All-in-all, today's oral contraceptives are much safer with regard to the incidence and severity of estrogen-linked clotting disorders as well as the suggested cumulative impact of more "lipid friendly" progestins that maintain the potentially advantageous high density lipoprotein cholesterol levels in circulation.

U.S. Patent 4,390,531 teaches a triphasic regimen in which each phase uses about 20-40 mcg ethinyl estradiol, phases 1 and 3 use 0.3-0.8 norethindrone and phase 2 doubles the amount of the norethindrone. These three phases consume 21 days of a 28 day treatment cycle. European published application 0 226 279 states that this regimen is associated with a high incidence of breakthrough bleeding and substitutes a three phase oral contraceptive regimen using a relatively low amount of ethinyl estradiol (10-50 µg) and a relatively high amount of norethindrone acetate (0.5-1.5 mg) in each phase provided that the amount of estrogen in any two phases is never the same. A "rest" phase of about 7 days is used in this regimen.

U.S. Patent 5,098,714 teaches an osmotic, oral dosage form. One "pill" is administered per day but the administration is, in effect, polyphasic. The dosage form is constructed such that it provides an initial pulse delivery of estrogen and progestin followed by prolonged delivery of estrogen.

European published patent application 0 253 607 describes a monophasic contraceptive preparation containing units having 0.008-0.03 mg of ethinyl estradiol and 0.025-0.1 mg of desogestrel (or equivalent) and a regimen where the preparation is administered over a 23-25 day period, preferably 24 days, followed by a 2-5 day pill-free period. The object of this regimen is to provide hormonal replacement therapy and contraceptive protection for the pre-menopausal woman in need thereof by supplying a low dose of an estrogen combined with a "very low dose of a progestogen."

In 1989, the accumulating data from the evolution of oral contraceptive pill formulations containing only 20-35 µg of estrogen per day spurred the Food and Drug Administration's Fertility and Maternal Health Drugs Advisory Committee to recommend indication of low dose oral contraceptives for healthy, non-smoking women even during the perimenopausal years, such as, for instance, ages 35-50. In Japan, oral contraceptives have recently been approved for the first time.

U.S. patent number 5,552,394 describes a method of female contraception which is characterized by a reduced incidence of breakthrough bleeding after the first cycle involves monophasioly administering a combination of estrogen and progestin for 23-25 consecutive days of a 28 day cycle in which the daily amounts of estrogen and progestin are equivalent to about 5-35 mcg of ethinyl estradiol and about 0.025 to 10 mg of norethindrone acetate, respectively and in which the weight ratio of estrogen to progestin is at least 1:45 calculated as ethinyl estradiol to norethindrone acetate.

In establishing a estrogen-progestin regimen for oral contraceptives, two principal issues must be confronted. First, efficacy must be maintained and second, there must be avoidance of further erosion in the control of endometrial bleeding. In general, even the lowest dose oral contraceptive products commercially available have demonstrated efficacy but the overall instances of bleeding control problems has increased as the doses were reduced, as manifest both in breakthrough bleeding (untimely flow or spotting) or withdrawal amenorrhea during the "pill free" week (expected menses).

It is the object of the present invention to provide a new estrogen-progestin combination and/or regimen for oral contraceptive use which maintains the efficacy and provides enhanced control of endometrial bleeding, the continuous use of which leads to amenorrhea (a lack of vaginal bleeding). The regimen enhances compliance by eliminating stop/start and also reduces or eliminates vaginal blood loss in patients, thereby reducing or eliminating the chances of anemia. Eliminating menstrual intervals can enhance lifestyles and convenience. This and other objects of the invention will become apparent to those skilled in the art from the following detailed description.

U.S. patent number 5,898,032 describes a method of female contraception which involves administering, preferably monophasicly, a combination of estrogen and progestin for 60-110 consecutive days followed by 3-10 days of no administration, in which the daily amounts of the estrogen and progestin are equivalent to about 5-35 mcg of ethinyl estradiol and about 0.025-10 mg of norethindrone acetate, respectively. It has now been found that the continuous administration of the combination can be extended to greater than one year. The duration determined by the patients medical condition and/or needs with efficacy being maintained and the induction of amenorrhea (lack of bleeding) avoids the medical problem of anemia, Moreover, symptoms which are generally attributed to hormone withdrawal (such as headaches, irritability, sleeplessness, cramps, and pelvic pressure, among others) can be avoided. Relative to women having natural or oral contraceptive-induced menstruation, the incidence of endometrial cancer will be reduced. Administration of agents to control endometriosis for this long period of time is known but this is the first time an oral contraceptive protocol of this length has been found.

### SUMMARY OF THE INVENTION

This invention relates to a method of female contraception which is characterized by eliminating withdrawal menses. More particularly, it relates to a method of female contraception which involves administering, preferably monophasicly, a combination of estrogen and progestin continuously for more than a year in which the daily amounts of the estrogen and progestin are equivalent to about 5-35 mcg of ethinyl estradiol and about 0.025-10 mg of norethindrone acetate, respectively.

### DESCRIPTION OF INVENTION

In accordance with the present invention, a woman in need of contraception is administered a combined dosage form of estrogen and progestin, preferably monophasicly, continuously for more than a year in which the daily amounts of estrogen and progestin are equivalent to about 5-35 mcg of ethinyl estradiol and about 0.025 to 10 mg of norethindrone acetate, respectively. The time duration is preferably at least about 1,1 years, more preferably at least about 1.25 or about 1,5 years, and can continue for about 5 years or more.

The preferred progestin and estrogen are norethindrone acetate and ethinyl estradiol, although other estrogens and progestins can be employed. The weight ratio of these two active ingredients is between 10 and 50, depending on the potency of the progestin. For example, levonorgestrel is believed to be approximately 10 times more potent than norethindrone or its acetate, so the weight in micrograms administered would be multiplied by 10 to calculate the progestin to estrogen ratio, The preferable amount of ethinyl estradiol is about 10-30 mcg and the preferable amount of the norethindrone acetate is about 0.25-1.5 mg. Other estrogens vary in potency from ethinyl estradiol. For example, 30 mcg of ethinyl estradiol is roughly equivalent to 60 mcg of mestranol or 2,000 mg of 17 β-estradiol. Likewise, other progestins vary in potency from norethindrone acetate. Thus, 3.5 mg of norethindrone acetate is roughly equivalent to 1 mg of levonorgestrel or desogestrel and 3-ketodesogestrel and about 0.7 mg of gestodene. The values given above are for the ethinyl estradiol and the norethindrone acetate and if a different estrogen or progestin is employed, an adjustment in the amount based on the relative potency should be made. The correlations in potency between the various estrogens and progestins are known.

Other useable estrogens include the esters of estradiol, estrone and ethinyl estradiol such as the acetate, sulfate, valerate or benzoate, conjugated equine estrogens, agnostic anti-estrogens, and selective estrogen receptor modulators. The estrogen is administered in the conventional manner by any route where it is active, for instance orally or transdermally. Most estrogens are orally active and that route of administration is therefore preferred. Accordingly, administration forms can be tablets, dragees, capsules or pills which contain the estrogen (and preferably the progestin) and a suitable pharmaceutically acceptable carrier,

Pharmaceutical formulations containing the progestin and a suitable carrier can be solid dosage forms which includes tablets, capsules, cachets, pellets, pills, powders or granules; topical dosage forms which includes solutions, powders, fluid emulsions, fluid suspensions, semi-solids, ointments, pastes, creams, gels or jellies, foams and controlled release depot entities; and parenteral dosage forms which includes solutions, suspensions, emulsions or dry powder comprising an effective amount of progestin as taught in this invention. It is known in the art that the active ingredient, the progestin, can be contained in such formulations in addition to pharmaceutically acceptable diluents, fillers, disintegrants, binders, lubricants, surfactants, hydrophobic vehicles, water soluble vehicles, emulsifiers, buffers, humectants, moisturizers, solubilizers, preservatives and the like, The means and methods for administration are known in the art and an artisan can refer to various pharmacologic references for guidance. For example, "Modem Pharmaceutics", Banker & Rhodes, Marcel Dekker, Inc. 1979; "Goodman & Gilman's The Pharmaceutical Basis of Therapeutics", 6th Edition, MacMillan Publishing Co., New York 1980 can be consulted.

The pharmaceutical formulations may be provided in kit form containing sufficient tablets intended for ingestion on successive days continuously to be obtained in amounts sufficient to insure continuous use until another supply can be obtained or the patient desires to discontinue the method. For example, medication could be supplied in packets of 30, 60 or 90, etc. depending on the patients desires as to how much medication she wants available.

In order to further illustrate the present invention, specific examples are set forth below. It will be appreciated, however, that these examples are illustrative only and are not intended to limit the scope of the invention.

### EXAMPLE 1

A study is carried out at a fully accredited animal research facility which complies through its animal care and use committee with the review standards set forth in the National Institute of Health's "Guide for Care and Use of Laboratory Animals", the Public Health Services' "Principles for the Care and Use of Laboratory Animals", and the United States Department of Agriculture's Implementation Regulations of the 1985 Amendments for the Animal Welfare Act.

Ten adult female cynomolgus monkeys (Macaca fasicularis) having regular presumably ovulatory menstrual cycles (28.9 ± 3.1 days for the month prior to study entry) are selected. Their duration of spontaneous menses is 3.4 ± 1.4 days. Mean body weight of the monkeys is 3.5 ± 1.1 kg (X ± SEM). They are housed individually in a controlled environment (12 hours of light and 23 °C). Their diet is a commercial primate food (Purina, St. Louis, MO) with water *ad libitum*.

The monkeys are divided at random into two groups (N=5 each). The studies begin with spontaneous menstruation in a pretreatment control cycle. At the onset of the next spontaneous menses, alternatively, they are assigned to receive on cycle day one an ultra low dose oral contraceptive continuously for more than one year. The study concludes with each group of primates being followed during a post-treatment spontaneous ovarian menstrual cycle.

Femoral blood is collected weekly and the serum frozen for subsequent RIA of estradiol, progesterone, FSH and LH in the pretreatment, treatment, and post-treatment phase. s Bleeding profiles are kept by daily vaginal swabs, indicating spontaneous menstruation, withdrawal bleeding, breakthrough bleeding, or amenorrhea. Breakthrough bleeding is defined as detectable blood in the vagina outside of the first 8 days after the last dose of oral contraceptive or the onset of spontaneous menses in non-treatment cycles.

Since the objective is to test an ultra low dose oral contraceptive, the medication is adjusted to fit the smaller (than human) body weight of these laboratory primates, The dose of ethinyl estradiol is 1.2 µg/day, while the dose of norethindrone acetate is 0.06 mg/day. This "in-house" reformulation is achieved by grinding to powder a commercially available monophasic pill (Loestrin 1/20, Parke Davis, Moms Plains, NJ), which originally contained 1 mg of norethindrone acetate and 20 µg of ethinyl estradiol per tablet, contained in a conventional 21 day pack along with 7 iron-containing placebos.

### EXAMPLES 2 - 5

The example 1 procedure is repeated using the following combinations of estrogen and progestin:

| Example | Estrogen | Progestin |
|---|---|---|
| 2 | mestranol | levo-norgestrel |
| 3 | 17-beta-estradiol | 3-keto-desogestrel |
| 4 | ethinyl estradiol | desogestrel |
| 5 | mestranol | gestodone |

Application of the compounds, compositions and methods of the present invention for the medical or pharmaceutical uses described can be accomplished by any clinical, medical, and pharmaceutical methods and techniques as are presently or prospectively known to those skilled in the art. It will therefore be appreciated that the various embodiments which have been described above are intended to illustrate the invention and various changes and modifications can be made in the inventive method without departing from the spirit and scope thereof.

## Claims

1. A method of female contraception which comprises monophasicly administering a combination of estrogen and progestin continuously for more than a year, in which the daily amounts of estrogen and progestin are equivalent to about 5-35 mcg of ethinyl estradiol and about 0.025 to 10 mg of norethindrone acetate, respectively,

2. The method of claim 1 in which the daily amount of estrogen is equivalent to about 10 to 30 mcg of ethinyl estradiol.

3. The method of claim 2 in which the daily amount of progestin is equivalent to 0.25-1.5 mg of norethindrone acetate.

4. The method of claim 3 in which the combination is administered for at least 370 consecutive days.

5. The method of claim 4 in which the estrogen is ethinyl estradiol.

6. The method of claim 5 in which the progestin is norethindrone acetate.

7. The method of claim 1 in which the daily amount of progestin is equivalent to 0.25-1.5 mg of norethindrone acetate.

8. The method of claim 1 in which the combination is administered for at least 370 consecutive days.

9. The method of claim 1 in which the estrogen is ethinyl estradiol.

10. The method of claim 1 in which the progestin is norethindrone acetate.

11. The method of claim 1 in which the daily amount of estrogen is up to 30 mcg of ethinyl estradiol.
